# EUROPÄISCHE PATENTSCHRIFT

(11) **EP 0 262 522 B1**
(45) Veröffentlichungstag und Bekanntmachung des Hinweises auf die Patenterteilung: **05.05.1993**
(21) Anmeldenummer: 87113626.3
(22) Anmeldetag: 17.09.1987
(51) Int. Cl.: A61B 6/14, A61B 6/06

(54) **Zahnärztliche Röntgendiagnostikeinrichtung**
Diagnostic dental X-ray apparatus
Appareil de radiodiagnostic dentaire

(30) Priorität: 30.09.1986 DE 3633252
(43) Veröffentlichungstag der Anmeldung: 06.04.1988
(73) Patentinhaber: SIEMENS AKTIENGESELLSCHAFT, 80333 München (DE)
(72) Erfinder: Adler, Rolf, Dipl.-Ing.(FH), D-6142 Bensheim-Fehlheim (DE); Heubeck, Erich, D-6140 Bensheim (DE); Müther, Manfred, D-6140 Bensheim (DE)

(56) Entgegenhaltungen:
- WO-A-84/03033
- DE-A- 2 058 423
- DE-A- 2 520 179
- US-A- 3 502 878
- US-A- 3 643 095
- US-A- 3 875 411
- US-A- 3 986 034
- US-A- 4 195 229

## Beschreibung

Die Erfindung bezieht sich auf eine zahnärztliche Röntgendiagnostikeinrichtung zur Erstellung von einerseits Panorama-Schichtaufnahmen vom Kiefer und andererseits Fernaufnahmen vom Schädel eines Patienten, enthaltend eine objektnahe erste Filmkassettenhalterung (5) zur Aufnahme von Filmkassetten (11) zur Erstellung der Panorama-Schichtaufnahmen und eine objektferne zweite Filmkassettenhalterung (44) zur Aufnahme von Filmkassetten (45) zur Erstellung der Fernaufnahmen, wobei die erste Filmkassettenhalterung (5) so ausgebildet ist, daß sie bei Fernaufnahmen aus den Strahlengang der Strahlenquelle (16) bringbar ist, und enthaltend ferner eine Halterung (20) für eine zwischen Strahlenquelle (16) und Objekt vorgesehene Blende zur Begrenzung des Strahlenbündels, welche zum Zwecke einer Änderung des Filmkassettenformates (A, B, C) gegen eine andere mit einer anderen Blendenöffnung wechselbar gehaltert ist.

Bei panorama-Röntgendiagnostikgeräten, mit denen Schichtaufnahmen vom Kiefer eines Patienten erstellt werden können, besteht häufig die Möglichkeit, mit Hilfe einer zusätzlichen, adaptierbaren Kopfhalterung Aufnahmen vom gesamten Schädel eines Patienten (sog. Ceph Fernaufnahmen) erstellen zu können. Bei einem Wechsel von Schichtaufnahme auf Ceph-Aufnahme ist es aus Strahlenschutzgründen notwendig, das Strahlenbündel der Primärblende auf das Kassettenformat zu begrenzen. Zu diesem Zweck sind bei einem bekannten Gerät (ORTHOPANTOMOGRAPH 10 - ORTOCEPH 10) für Schichtaufnahmen einerseits und Ceph-Aufnahmen andererseits zwei mit verschiedenen Blendenöffnungen versehene Primärblenden vorhanden, die ausgewechselt werden müssen, wenn von Schichtaufnahme auf Ceph-Aufnahme und umgekehrt umgestellt wird.

Darüber hinaus besteht neben der Möglichkeit, alternativ zwischen Schichtaufnahme und Ceph-Aufnahme wählen zu können, auch der Wunsch, unterschiedlich große Bereiche vom Objekt, also beispielsweise Ausschnitte vom Schädel oder vom Kiefer, aufnehmen zu können. Hierzu kann es angebracht sein, das Kassettenformat zu wechseln und aus Strahlenschutzgründen das Strahlenbündel auf das gewählte Kassettenformat zu begrenzen.

Aus der früheren, jedoch nicht vorveröffentlichten europäischen Patentanmeldung EP-A-0 236 790 ist zahnärztliches Röntgendiagnostikgerät bekannt, das ein Mehrfachblendenteil mit einer Vielzahl von Unterschiedlichen Blendenöffnungen umfasst. Das Mehrfachblendenteil ist um eine gemeinsame Achslagerung derart schwenkbar gehaltert, daß sich die Blendenöffnungen programmgesteuert jeweils auf den Strahlengang der Strahlenquelle ausrichten lassen.

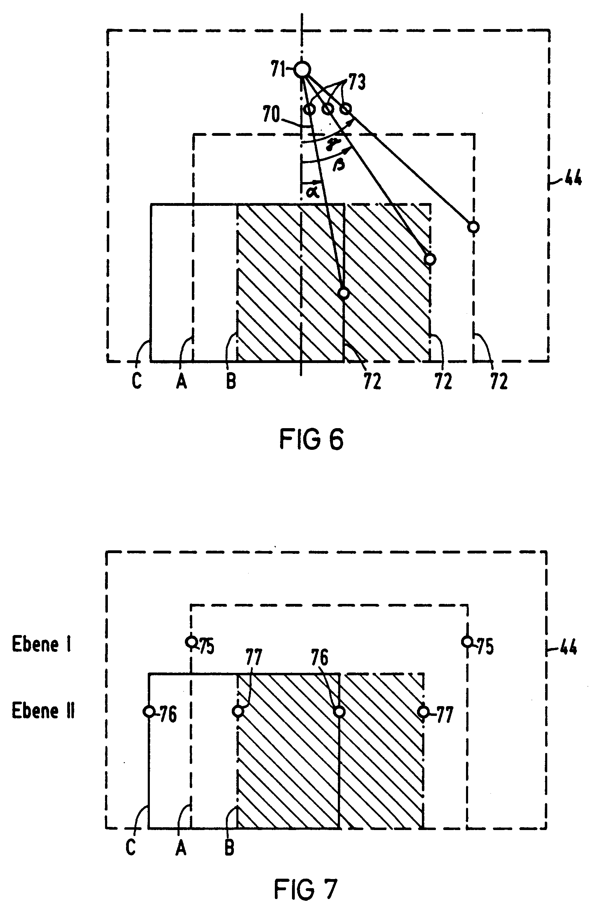

Aus der WO-A-84 03 033 ist eine Blendenanordnung für sogenannte intraorale Röntgengeräte bekannt, bei denen die Strahlenquelle intraoral, also im Patientenmund, und der Filmhalter mit dem zu belichtenden Film extraoral angeordnet sind. Der Filmhalter gestattet es, einzelne unterschiedliche Blendenöffnungen aufzuweisende Blendeneinsätze (7a, 7b, 7c) in Form von schmalen rechteckigen Metallplatten (Fig. 5) einzusetzen. Die Blendeneinsätze werden in quer zur Strahlenaustrittsöffnung angeordneten Führungselementen eingesetzt und können entlang dieser in mehreren Positionen gerastet werden. Die Rastung dient hier zur Erstellung von sogenannten stereografischen Aufnahmen, bei denen mehrere Bilder vom durchstrahlten Objekt aus verschiedenen Positionen relativ zur Strahlenquelle erstellt werden.

Die bekannte Blendenanordnung ist nicht für eine Anwendung bei einer Röntgendiagnostikeinrichtung der erfindungsgemäßen Gattung vorgesehen; sie enthält weiterhin auch kein Mehrfachblendenteil mit unterschiedlichen Blendenöffnungen, die durch eine Verstelleinrichtung auf den Strahlengang der Strahlenquelle ausgerichtet werden können.

Aus der US-36 43 095 ist eine automatische Verschlußblendensteuerung für ein Röntgenuntersuchungsgerät bekannt, welches, wie beispielsweise aus US-39 86 034 bekannt, einen Untersuchungstisch mit Patientenlagerungsplatte und eine darunter verstellbar angeordnete Kassettenlade für die aufzunehmende Filmkassette enthält. Oberhalb des Untersuchungstisches ist in einem definierten Abstand die Röntgenstrahlenquelle mit Primärblende angeordnet. Die Primärblende wird in der Regel durch zwei Paar verstellbarer Blendenlamellen gebildet, deren einander gegenüberliegende Kanten die Blendenöffnung bestimmen. Die Blendenöffnung und damit der von der Strahlenquelle bestrahlte Bereich können mit Hilfe von Steuermitteln selbsttätig auf die Größe der verwendeten Filmkassettte eingestellt werden. Bei dem Röntgenuntersuchungsgerät entsprechend der US-39 86 034 sind im Bereich der Kassettenaufnahme Sensormittel in Form von Spannbacken vorgesehen, die die Größe der Filmkassette abtasten und entsprechend dem erhaltenen Signal die Blendenlamellen der Primärblende verstellen.

Der im Anspruch 1 angegebenen Erfindung liegt die Aufgabe zugrunde, eine Röntgendiagnostikeinrichtung der eingangs genannten Gattung dahingehend zu verbessern, daß es unter Vermeidung einer umständlichen Handhabung möglich ist, alternativ Kieferschichtaufnahmen, Schädelaufnahmen oder Aufnahmen von Teilbereichen dieser Objekte mit unterschiedlichen Filmkassetten erstellen zu können, wobei sichergestellt sein soll, daß bei einem Wechsel der Aufnahmeart und innerhalb einer Aufnahmeart bei einem Wechsel des Filmkassettenformates stets die dem gewählten Kassettenformat bzw. dem gewählten Objekt entsprechende Primärblende zum Einsatz kommt, so daß keine falsche, möglicherweise unzulässig hohe Strahlendosis abgegeben wird.

Die Logikschaltung zur Steuerung der Strahlenquelle ist vorteilhafterweise so ausgelegt, daß die Signale aus der Sensoranordnung, welche dem gewählten Filmkassettenformat entsprechen, mit Signalen aus einer weiteren Sensoranordnung verglichen werden, die dem Mehrfachblendenteil zugeordnet ist. In einer Vergleichslogik wird so überprüft, ob einem gewählten Filmkassettenformat bzw. dem gewählten Objekt auch die richtige Blende zugeordnet ist; ist dies nicht der Fall, bleibt die Steuereinheit verriegelt, also inaktiv. Die Freigabe von Strahlung erfolgt erst dann, wenn Filmkassettenformat und Blende bzw. Blendenöffnung zueinander passen. Das Verstellen der Blende kann dabei sowohl von Hand als auch motorisch erfolgen. Im Falle einer motorischen Verstellung der Blende ist es vorteilhaft, einen Träger für das Mehrfachblendenteil vorzusehen und den Träger direkt in Abhängigkeit vom gewählten Filmkassettenformat zu steuern.

Die Sensoranordnung kann aus einer optischen Anordnung bestehen, mit beispielsweise Leuchtdioden und Fotoelementen oder Reflex-Lichtschranken, die in der Filmkassettenhalterung entsprechend den vorgesehenen Kassettenformaten angeordnet sind. Vorteilhafterweise ist die Sensoranordnung so angeordnet, daß die Außenkanten der Filmkassetten in wenigstens einer der beiden Dimensionen (Breite oder Höhe) erfaßt werden.

Die Sensoranordnung kann auch aus optischen oder aus einer Kombination von optischen elektrischen und mechanischen Mitteln bestehen. Gemäß einer weiteren vorteilhaften Ausgestaltung der Erfindung wird vorgeschlagenen, wenigstens einen schwenkbar gelagerten Tasthebel vorzusehen, dessen eines Ende an einer das Kassettenformat bestimmenden Referenzfläche an der Kassette anliegt und dessen Schwenkstellungen bei eingeschobener Kassette über z.B. eine Lichtschranke erfaßt wird. Der schwenkbar gelagerte Tasthebel kann alternativ auch mit einem Potentiometer gekoppelt sein, wodurch sich in den den unterschiedlichen Filmkassettenformaten entsprechenden Schwenkstellungen unterschiedliche Widerstandswerte ergeben, deren analoge Signale über einen AD-Wandler in digitale Signale umgewandelt werden können.

Alternativ zu Potentiometern können auch optische Winkelgeber vorgesehen sein, in Form z.B. mit dem Tasthebel verbundenen Sektorscheiben, die sich zwischen Leucht- und Fotodioden bewegen.

Die von der Sensoranordnung gewonnenen Signale, die einerseits zu erkennen geben, daß eine Filmkassette in die Halterung eingesetzt ist und andererseits eine Größe für das gewählte Format liefern, können in konventioneller Verarbeitungstechnik einem Komparator zugeführt werden, der die ermittelten Werte mit gespeicherten Werten vergleicht und ein Ausgangssignal an eine Steuereinheit abgibt, welche dazu vorgesehen ist, das Mehrfachblendenteil entsprechend zu verstellen.

Die Aufbereitung und der Vergleich der Signale kann vorteilhafterweise mittels integrierter Schaltungstechnik erfolgen.

Das Mehrfachblendenteil kann unterschiedlich aufgebaut sein. Vorteilhafterweise besteht es aus einem Träger mit allen vorkommenden Blendenöffnungen, der drehbar oder längsverschiebbar angeordnet ist, wobei ein Verstellmotor die Blendenöffnungen in der erwähnten Weise in die jeweils erforderliche Position bringt.

Das Mehrfachblendenteil kann vorteilhafterweise die bereits erwähnte weitere Sensoranordnung enthalten, welche blendenspezifische Informationen an eine Vergleichslogik meldet.

Weitere vorteilhafte Ausgestaltungen und Weiterbildungen der Erfindung sind den Unteransprüchen und der nachfolgenden Beschreibung mehrerer Ausführungsbeispiele zu entnehmen.

Es zeigen:
Figur 1 ein Röntgendiagnostikgerät der erfindungsgemäßen Gattung in schaubildlicher Darstellung,
Figur 2 den Röntgenstrahler des Gerätes nach Figur 1 teilweise aufgeschnitten in schaubildlicher Darstellung,
Figur 3 eine Ausführungsform des Mehrfachblendenteils nach Figur 1 in schaubildlicher Darstellung,
Figur 4 eine an das Gerät nach Figur 1 adaptierbare Filmkassettenhalterung und Kopfpositioniereinrichtung zur Erstellung von Fernröntgenaufnahmen in schaubildlicher Darstellung,
Figur 5 die Einrichtung nach Figur 4 in Frontansicht, teilweise im Schnitt,
Figuren 6 und 7 mehrere Varianten einer Sensoreinrichtung zur Erfassung des Filmformates in schematischer Darstellung,
Figuren 8 und 9 Prinzipschaltbilder, die die Signalverarbeitung der Sensoreinrichtung veranschaulichen.

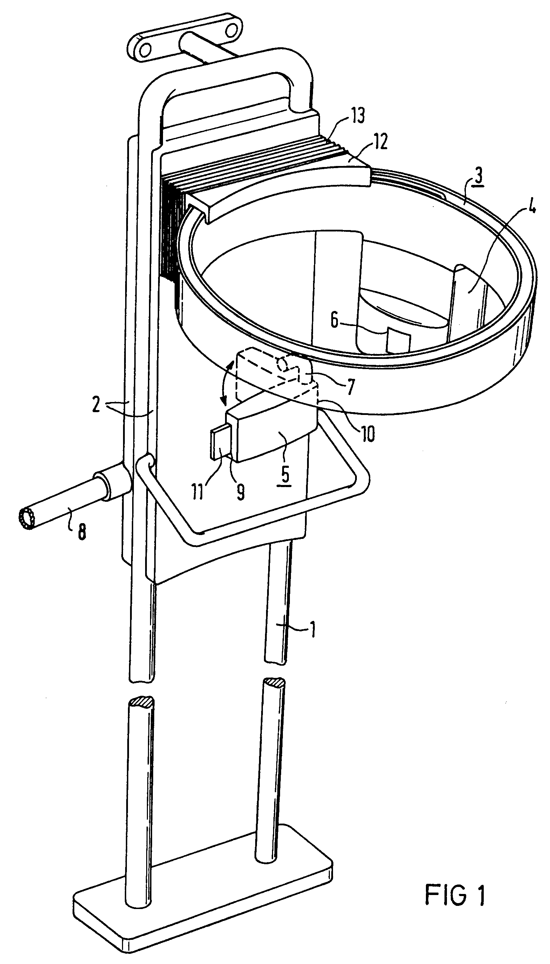

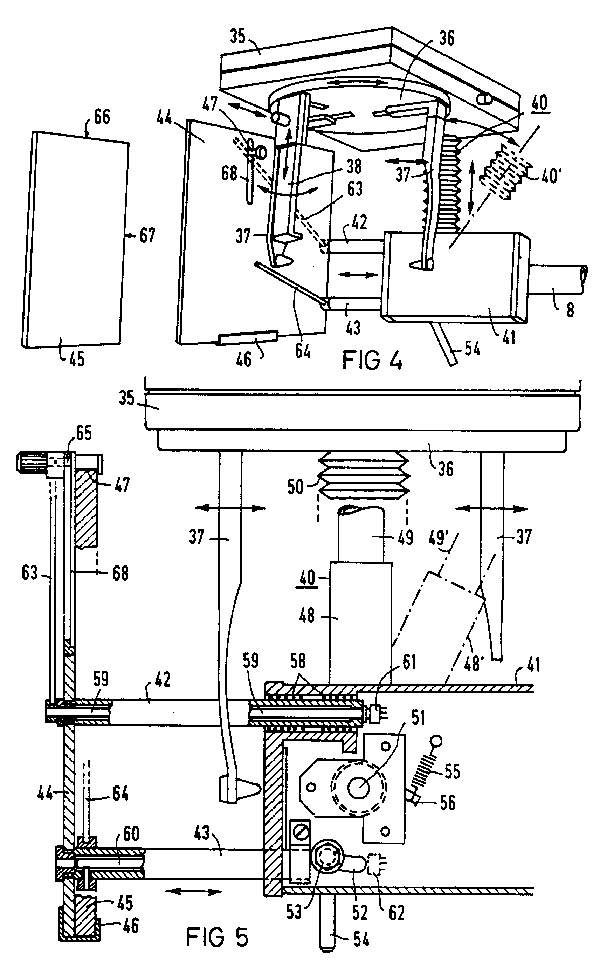

Die Figur 1 zeigt in einer schaubildlichen Darstellung eine mögliche Ausführungsform eines zahnärztlichen Röntgendiagnostikgerätes der erfindungsgemäßen Gattung. Das Gerät enthält ein aus Zwei Standrohren gebildetes Stativ 1, an dem ein Laufwagen 2 höhenverstellbar gehaltert ist. Am Laufwagen 2 ist eine allgemein mit 3 bezeichnete Dreheinheit in Form eines geschlossenen Ringes gehaltert, die einerseits einen Röntgenstrahler 4 und diametral dazu eine Filmkassettenhalterung 5 enthält. Während der Röntgenstrahler 4, dessen gehäuseseitige Strahlenaustrittsöffnung mit 6 bezeichnet ist, am Drehring 3 drehfest angeordnet ist, ist der Filmkassettenhalter 5 an einem abgewinkelten Tragarm 7 in Pfeilrichtung schwenkbar gehaltert. Der Filmkassettenhalter 5 kann so aus der gestrichelt eingezeichneten Gebrauchsstellung, die für normale Schichtaufnahmen geeignet ist, in die mit durchgehenden Linien gezeichnete Nichtgebrauchsstellung gebracht werden, die einerseits dem Hilfspersonel Positionieren des zwischen Strahlenquelle 4 und Filmkassettenhalter 5 anzuordnenden Patientenkopfes erleichtert und andererseits das Erstellen von Fernaufnahmen, sog. Ceph-Aufnahmen, ermöglicht. Um solche Fernaufnahmen machen zu können, muß in einer bestimmten Entfernung (ca. 1,5 m) vom Strahler 4 eine Kopfpositioniereinrichtung und Kassettenhalterung vorgesehen werden. Eine solche Kopfpositioniereinrichtung und Kassettenhalterung, welche mittels eines Tragrohres 8 am Laufwagen 2 gehaltert ist, zeigen Figuren 4 und 5.

Der Filmkassettenhalter 5, der zur Aufnahme normaler Schichtaufnahmen vorgesehen ist, enthält an beiden Stirnseiten schlitzförmige Ein- und Austrittsöffnungen 9, 10, über die die mit 11 bezeichnete Filmkassette eingeführt bzw. nach der Aufnaheme entnommen werden kann. Bei der verwendeten Filmkassette handelt es sich um eine flexible, mit Verstärkerfolie versehene Filmkassette, wie sie im Prinzip für intraorale Aufnahmen verwendet wird. Der Transport der Filmkassette erfolgt in nicht näher dargestellter Weise durch einen im Filmkassettenhalter 5 angeordneten elektromotorischen Antrieb.

Der Drehring 3 ist in einem Lagerteil 12 drehbar und gegenüber dem Laufwagen 2 auch schwenkbar gehaltert. Die hierzu erforderliche, nicht näher dargestellte Verstellmechanik befindet sich zwischen Laufwagen 2 und Drehring 3 und ist durch einen Faltenbalg 13 abgedeckt. Die Verstelleinrichtung ermöglicht es, durch entsprechende Steuerung von nicht dargestellten Verstellmotoren den Drehring und damit die Position der Röntgenstrahlenquelle 4 und Filmkassettenhalter 5 in jede beliebige, für den Umlauf um den Patientenkopf notwendige Position zu bringen. In Verbindung mit der Eigendrehbewegung, die der Drehring 3 auch noch um seine Mittelpunktachse ausführen kann und der im Kassettenhalter 5 verstellbaren Filmkassette kann so der Bewegungsablauf der gewünschten Aufnahme entsprechend gesteuert werden.

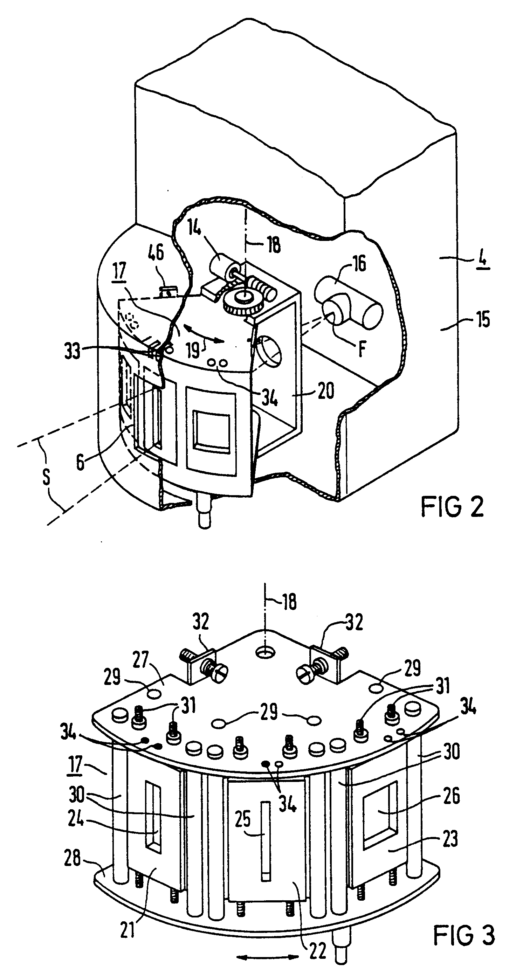

Die Figur 2 zeigt in einer schaubildlichen Darstellung den Röntgenstrahler 4 mit teilweise aufgebrochenem Gehäuse.

Im rückwärtigen Teil des mit 15 bezeichneten Gehäuses ist in bekannter Weise eine Röntgenröhre 16, also die eigentliche Strahlenquelle, angeordnet, von der aus allgemein mit S bezeichnete Strahlen austreten, die nach Durchstrahlen des Patientenkopfes den in der Filmkassette 11 angeordneten Film belichten. Der Fokus des von der Röhre 16 ausgehenden Strahlenbündels ist mit F bezeichnet. Im Strahlengang zwischen Strahlenquelle 16 und dem gehäuseseitigen Fenster 6 ist ein Mehrfachblendenteil 17 vorgesehen, welches mittels einer vertikalen Achslagerung 18 in Richtung des angegebenen Pfeiles 19 schwenkbar am Gehäuse des Strahlers gehaltert ist. Zur Lagerung des Mehrfachblendenteils 17 ist ein U-förmig ausgebildetes, mit einer entsprechenden Öffnung für den Durchtritt der Strahlung versehenes Halteteil 20 vorgesehen. Das Mehrfachblendenteil 17 wird mittels einer allgemein mit 14 bezeichnteten Antriebseinheit, die in bekannter Weise aus einem Schrittmotor mit Getriebe bestehen kann, angetrieben, wodurch, wie später noch näher erläutert, eine der mehreren Blendenöffnungen des Mehrfachblendenteils 17 auf den Strahlengang des Strahlers ausgerichtet werden kann.

Die Figur 3 zeigt in schaubildlicher Darstellung das Mehrfachblendenteil 17 als Einzelteil. Es enthält drei Blendeneinsätze 21, 22, 23 mit jeweils unterschiedlich großen Öffnungen 24, 25 und 26. Die Größe der Blendenöffnungen 24, 25 und 26 entspricht unterschiedlichen Filmformaten bei Ceph-Aufnahmen und/oder unterschiedlichen Aufnahmen bei Schichtaufnahmen. Es sei an dieser Stelle vermerkt, daß anstelle der gezeigten drei unterschiedlichen Blendenöffnungen noch weitere vorgesehen sein können. Im hier gezeigten Ausführungsbeispiel sind zur Aufnahme der Blendeneinsätze 21 bis 23 zwei etwa die Form eines Kreissektors aufweisende Platten 27, 28 vorgesehen, die durch mehrere, senkrechte Haltebolzen 29 auf Abstand gehalten werden. Zur Halterung der Blendeneinsätze sind im peripheren Bereich der Platten 27, 28 weitere Haltebolzen 30 vorgesehen, die durch nicht gezeigte Sicherungsringe lösbar sind, wodurch ein rasches Wechseln der Blendeneinsätze möglich ist. Mit 31 und 32 sind Justier- bzw. Anschlagschrauben bezeichnet, mit denen die Blendeneinsätze sich auf den Zentralstrahl bzw. auf den Strahlengang des Strahlers 16 einstellen lassen bzw. mit denen sich das Mehrfachblendenteil exakt ausrichten läßt.

Das Mehrfachblendenteil 17 enthält eine Sensoranordnung die deren momentane Stellung und damit die Stellung der Blendenöffnungen 24, 25 und 26 in bezug auf die Strahlenquelle 16 erfaßt. Die Sensoranordnung wird im vorliegenden Ausführungsbeispiel gebildet durch zwei im Gehäuse 15 mittig angeordnete Lichtschranken 33 (Fig. 2) die mit den Blendeneinsätzen 21 bis 23 zugeordneten und an der oberen Platte 27 angeordneten, Lagemeldern 34 in Form von z.B. Vertiefungen, Bohrungen oder Erhebungen zusammenwirken. Die Lagemelder 34 haben unterschiedliche Kennungen, z.B. der Blende 23 zugeordnet zwei Bohrungen, der Blende 22 zugeordnet nur eine Bohrung und der Blende 21 zugeordnet überhaupt keine Bohrungen, so daß von den beiden Lichtschranken 33 eindeutig erkannt wird, welche Blende sich gerade im Strahlengang des Strahles 16 befindet.

Die Figur 4 zeigt in einer schaubildlichen Darstellung eine Kassettenhalte- und Kopfpositioniereinrichtung, wie sie zur Erstellung von Röntgenfernaufnahmen (Ceph-Aufnahmen) geeignet ist. Die Einrichtung enthält einen Schädelhalter 35, an dem in bekannter Weise ein Drehteller 36 drehbar gehaltert ist, an dem einerseits zwei diametral einander gegenüberliegende, in Pfeilrichtung verstellbare Ohrolivenhalter 37 und andererseits mit diesen in der Draufsicht etwa ein Dreieck bildendes Nasionabstützteil 38 angeordnet sind. Das Nasionabstützteil 38 ist in Höhe und Tiefe (in angegebener Pfeilrichtung) verstellbar sowie um eine Achslagerung schwenkbar am Drehteller 36 gehaltert.

Der Schädelhalter 35 ist mittels eines vertikal verlaufenden Tragteils 40 mit dem Tragrohr 8 verbunden. Mit 41 ist ein Gehäuse bezeichnet, an dem einerseits Tragrohr 8 und Tragteil 40 und andererseits zwei horizontale Tragstangen 42, 43 als Träger für eine Filmkassettenhalterung 44 gehaltert sind. Die beiden Tragstangen 42, 43 sind in der angegebenen Pfeilrichtung verstellbar im Gehäuse 41 gehaltert. Eine den Röntgenfilm enthaltende Filmkassette 45 wird senkrecht stehend in die Filmkassettenhalterung 44 eingelegt, wozu entsprechende Führungsglieder 46, 47 vorhanden sind.

Die Fig. 5 zeigt die Einrichtung nach Fig. 4 in Frontansicht teilweise im Schnitt. Das Tragteil 40 besteht aus zwei teleskopartig ineinander greifenden Rohren 48, 49, die durch einen gemeinsamen Faltenbalg 50 abgedeckt sind. Um verschiedene Ausziehlängen und damit eine Höhenverstellung des Schädelhalters 35 gegenüber dem Gehäuse 41 zu erzielen, ist eines der beiden Rohre mit einer Längsnut und die andere mit einer Bohrung versehen durch die eine Felststellschraube greift (nicht dargestellt). Neben der Höhenverstellbarkeit, die insbesondere dazu dient bei Spezialaufnahmen auch den letzten Halswirbel mit abzubilden, ist der gesamte Schädelhalter 35 noch um eine horizontale Achslagerung 51 schwenkbar, wie dies durch die gestrichelte Darstellung des Tragteils 40 in Fig. 4 (40') sowie der Teleskoprohre 48, 49 in Fig. 5 angegeben ist. Der Neigungswinkel ist begrenzt durch eine Nut 52 in einer Wandung des Gehäuses 41 und einen durch die Nut hindurchgreifenden Gewindebolzen 53, welcher das untere Ende des Telekkoprohrs 48 durchdringt und dort entsprechend befestigt ist. Am Gewindebolzen 53 ist ferner ein Spannhebel 54 aufgeschraubt, bei dessen Betätigung das Teleskoprohr 48 und somit das gesamte Tragteil 70 mit dem Gehäuse 41 verspannt und damit arretiert werden kann.

Damit der Schädelhalter 35 beim Öffnen des Spannhebels 54 seine Grundstellung beibehält, ist im Gehäuse 41 eine Zugfeder 55 vorgesehen, die einerseits mit dem Gehäuse 41 und andererseits mit einem mit einer Buchse winkelfest verbundenen, zur Drehachse 51 einen Hebelarm bildenden Abstützteil 56 verbunden ist.

Die beiden Tragstangen 42, 43 sind als Rohre ausgebildet und mittels Lager 58 leichtgängig geführt. Im Inneren der rohrförmigen Stangen 42, 43 sind Übertragungselemente 59, 60 in Form von ebenfalls Stangen oder Hülsen gelagert, deren eine Enden mit Winkelgeber in Form von Potentiometern 61, 62 und andere Enden mit Tasthebeln 63, 64 winkelsteif verbunden sind, von denen der mit 63 bezeichnete Tasthebel hinter, und der mit 64 bezeichnete Tasthebel vor der Filmkassettenhalterung 44 angeordnet ist. Die beiden Tasthebel 63, 64 sind Teil einer Sensoranordnung, mit der selbsttätig das vorgesehene Filmkassettenformat beim Aufschieben auf die Filmkassettenhalterung 44 bestimmt und ggf. auf einem Display angezeigt bzw. deren Signalen eine dem gewählten Filmkassettenformat zugehörige Primärblende automatisch in den Strahlengang zwischen Strahlenquelle und Objekt gebracht werden kann.

Aus Fig. 5 ist ersichtlich, daß der Tasthebel 63 einerseits mit der die Filmkassettenhalterung 44 durchdringenden Übertragungsstange 59 und andererseits mittels einer weiteren, die Filmkassettenhalterung durchdringender Querverbindung 65 welches das als Tastkopf wirkende Führungsglied 47 enthält verbunden ist. Während letzteres 47 auf einer die Oberkante des Filmkassettenformates bestimmenden Referenzfläche 66 (Fig. 4) aufliegt, liegt das freie Ende des Tasthebels 64 direkt an einer die Breite des gewählten Filmkassettenformats entsprechenden Referenzfläche 67 an. Der jeweilige Schwenkwinkel der beiden Tasthebel 63, 64 entsprechend der Anlage ihrer Enden an den Referenzflächen 66, 67 der aufgesetzten Filmkassette bestimmt das gewählte Filmkassettenformat. Damit sich das Führungsglied 47 unterschiedlich hohen Kassettenformaten anpassen kann, ist die Querverbindung 65 in einem lotrecht verlaufenden Schlitz 68 der Halterung 44 geführt.

Wie bereits erwähnt, können verschieden große Filmkassetten zur Anwendung kommen, z.B. solche wie in den Figuren 6 und 7 schematisch aufgezeigt, mit dem Format A mit den Abmessungen 24 x 30 cm oder mit dem Format B mit den Abmessungen 18 x 24 cm oder mit dem Format C, ebenfalls der Größe 18 x 24 cm, jedoch mit gegenüber dem Format B asymmetrischer Anordnung der Kassette relativ zum Kassettenhalter.

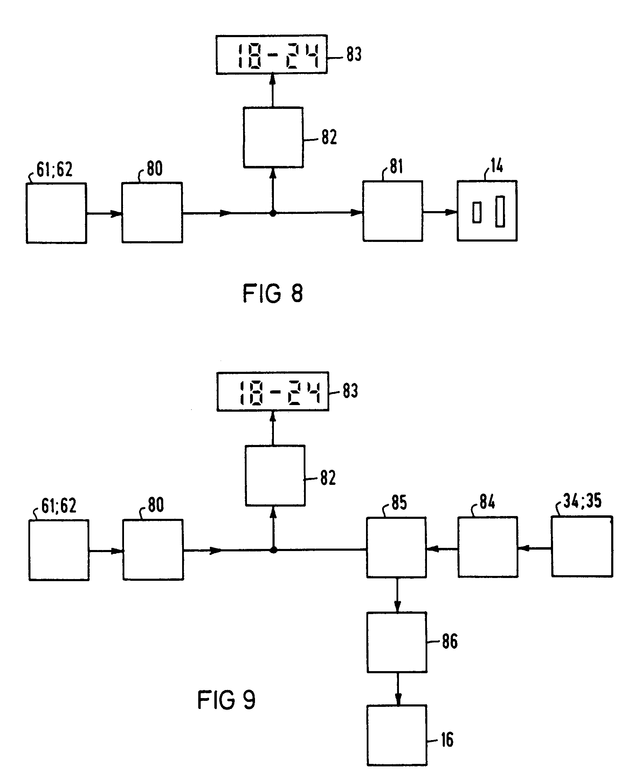

Wie aus der gezeigten Anordnung hervorgeht, wird mittels des Tasthebels 64 die Breite des Filmformates und mittels des Tasthebels 63 die Höhe des gewählten Filmformates erfaßt. Ein bestimmtes Filmkassettenformat ist demnach bestimmt durch einen bestimmten Schwenkwinkel der Tasthebel 63, 64. Wird also eine Filmkassette eines bestimmten Formates (A, B, C) auf die Halterungsplatte 44 aufgesetzt, so läßt sich über die Stellung der beiden Tasthebel 63, 64 das Filmkassettenformat bestimmen, indem über die beiden Potentiometer 61, 62 analoge, den Schwenkwinkeln der Tasthebel entsprechende Signale erzeugt werden, die nach entsprechender Aufbereitung und Auswertung entweder die Größe des Kassettenformates direkt an einem Display (Pos. 83 in Fig. 8 und 9) anzeigen und oder dazu vorgesehen werden, den Stellmotor 14 für das in den Figuren 2 und 3 beschriebene Mehrfachblendenteil anzusteuern, wodurch nach Einschieben der Filmkassette die diesem Kassettenformat zugehörige Blendenöffnung der Primärblende automatisch in den Strahlengang gebracht werden kann.

Die Figuren 6 und 7 zeigen weitere vorteilhafte Ausführungsform einer Sensoranordnung. Im Gegensatz zu der zuvor beschriebenen Ausführung ist bei der Ausführung nach Fig. 6 nur ein Tasthebel 70 vorhanden, der in der Filmhalterungsplatte 44 mittels einer nicht näher bezeichneten Lagerung schwenkbar gelagert ist, wobei auch hier der Tasthebel 70 mit einem mit der Pos. 71 angedeuteten Potentiometer verbunden ist. Im Gegensatz zu der zuvor erläuterten Ausführungsform liegt der Tasthebel 70 nur an einer, die Breite der eingeschobenen Kassette bestimmenden Referenzfläche 72 der drei Kassettenformate A, B, C an. Der durch die Schwenkstellung ermittelte Widerstandswert am Potentiometer 71 entsprechend den Schwenkstellungen mit den Schwenkwinkeln α, β und γ stellt ein Maß für das gewählte Filmformat dar. Die gewonnenen analogen Signale können in einem AD-Wandler digitalisiert und in einem Display angezeigt werden. Alternativ oder zusätzlich können die Signale dazu verwendet werden, die Verstelleinrichtung für das Mehrfachblendenteil anzusteuern, wie dies in den vereinfachten Schaltbildern nach Figuren 8 und 9 dargestellt ist. Nachdem der Tasthebel 70 bei dem hier gezeigten Ausführungsbeispiel in die Kassette hineinragt, ist für den Tasthebel ein Material zu wählen, welches auf dem Film keinen Schatten abbildet.

Denkbar ist es auch, anstelle der Potentiometer zur Erfassung der Winkelstellungen α, β, γ des Tasthebels 70 optische Sensorelemente 73 z.B. in Form von Reflexlichtschranken oder Leuchtdioden und Fotoelementen vorzusehen, zwischen denen der Tasthebel sich bewegt.

Eine weitere Ausführungsform einer Formaterfassung zeigt die Figur 7. Bei dieser Ausführungsform sind in einer ersten horizontalen Ebene (EI) für das größte Filmkassettenformat A ein erstes Sensorpaar und für die beiden niedrigeren aber gleich hohen Filmformate B und C zweite und dritte Sensorpaare 76 und 77 in einer zweiten Ebene (EII) vorgesehen. Die Sensorpaare 75 bis 77 sind an der Filmkassettenhalterungsplatte 44 so angeordnet, daß sie die äußeren Begrenzungen der eingelegten Filmkassette bestimmen. Die von den Sensoren gewonnenen Signale können, wie bereits erläutert, ausgewertet und zur Anzeige und/oder zur Steuerung der Antriebseinheit zur Verstellung der Primärblenden herangezogen werden.

Die Figuren 8 und 9 zeichen in vereinfachter Darstellung die Signalverarbeitung unter Zugrundelegung der Sensoranordnung gemäß Ausführungsbeispiel nach Figuren 1 bis 5.

Gemäß Figur 8 werden die Signale aus den Sensoren 61, 62, welche das Format der Filmkassette repräsentieren, über eine logische Aufbereitungsschaltung 80 einerseits einer Steuereinheit 81 zugeführt, mit der der Verstellmotor 14 (Figur 2) für das Mehrfachblendenteil angesteuert wird, und andererseits über einen Anzeigentreiber 82 einem Display 83 zugeführt, an dem Kassettenformat optisch angezeigt wird.

Im einfachsten Fall kann die Signalverarbeitung über die Steuereinheit 81 entfallen, d.h. dann wird lediglich das Filmformat entsprechend der von der Bedienperson eingelegten Filmkassette am Display angezeigt; im anderen Fall kann zusätzlich oder alternativ über den Stellmotor 14 gleich die richtige Blende in den Strahlengang positioniert werden.

Die Signalverarbeitung nach Figur 9 ist bezüglich des das Filmkassettenformat bestimmenden und anzeigenden Teils wie beschrieben; zusätzlich werden die Signale aus den Blendenpositionssensoren 34, 35 (Figur 2) nach einer logischen Aufbereitung (Pos. 84) eienr Vergleichslogik 85 zugeführt, in der die Signale mit jenen welche das Filmkassettenformat repräsentieren, verglichen werden. Stimmen die Signale von gewähltem Filmkassettenformat und zugeordneter Blende überein, so wird über eine Steuereinheit 86 der Röntgenstrahler 16 (Figur 2) eingeschaltet, im anderen Falle bleibt der Strahler so lange verriegelt, bis Übereinstimmung herbeigeführt ist, d.h. bis Blende und Filmkassette zueinander passen.

## Patentansprüche

1. Zahnärztliche Röntgendiagnostikeinrichtung zur Erstellung von einerseits Panorama-Schichtaufnahmen vom Kiefer und andererseits Fernaufnahmen vom Schädel eines Patienten, enthaltend eine objektnahe erste Filmkassettenhalterung (5) zur Aufnahme von Filmkassetten (11) zur Erstellung der Panorama-Schichtaufnahmen und eine objektferne zweite Filmkassettenhalterung (44) zur Aufnahme von Filmkassetten (45) zur Erstellung der Fernaufnahmen, wobei die erste Filmkassettenhalterung (5) so ausgebildet ist, daß sie bei Fernaufnahmen aus den Strahlengang der Strahlenquelle (16) bringbar ist, und enthaltend ferner eine Halterung (20) für eine zwischen Strahlenquelle (16) und Objekt vorgesehene Blende zur Begrenzung des Strahlenbündels, welche zum Zwecke einer Änderung des Filmkassettenformates (A, B, C) gegen eine andere mit einer anderen Blendenöffnung wechselbar gehaltert ist, **dadurch gekennzeichne t**, daß die zweite Filmkassettenhalterung (44) eine Sensoranordnung (59 bis 65; 70, 71, 73; 75 bis 77) aufweist, welche die Lage und das Format (A, B, C) der in die Halterung (44) eingesetzten Filmkassette (45) erfaßt, daß die das Strahlenbündel begrenzende Blende als Mehrfachblendenteil (17) mit mehreren Blendenöffnungen (24 bis 26) ausgebildet ist, daß das Mehrfachblendenteil (17) mittels einer Verstelleinrichtung (14) so steuerbar ist, daß die Blendenöffnungen jeweils auf den Strahlengang der Strahlenquelle ausrichtbar sind, und daß eine Logikschaltung (80) vorgesehen ist, welche die aus der Sensoranordnung gewonnenen Signale aufbereitet und einer Steuereinheit (81, 86) zuführt, welche die Verstelleinrichtung (14) für das Mehrfachblendenteil (17) in Abhängigkeit vom gewählten Filmformat steuert.

2. Röntgendiagnostikeinrichtung nach Anspruch 1, **dadurch gekennzeichnet**, daß eine weitere Sensoranordnung (34, 35) am Mehrfachblendenteil (17) vorhanden ist, welche der Stellung der einzelnen Blenden (21 bis 23) entsprechende Signale liefert, die einer Vergleichslogik (85) zugeführt werden, in die auch die Signale aus der das Filmkassettenformat bestimmenden Sensoranordnung (61, 62; 70, 71; 75 bis 77) eingegeben werden, und daß mit dem Ausgangssignal der Vergleichslogik (85) die Strahlenquelle (16) gesteuert wird.

3. Röntgendiagnostikeinrichtung nach Anspruch 1, **dadurch gekennzeichnet**, daß eine Sensoranordnung mit optischen Elementen (70, 71; 73, 75 bis 77), wie beispielsweise Leuchtdioden und Fotoelementen oder Reflexlichtschranken, vorgesehen ist, und die Elemente an die Breite und/oder Höhe des Filmkassettenformates (A, B, C) bestimmenden Referenzflächen (66, 67) der Filmkassettenhalterung (44) angeordnet sind.

4. Röntgendiagnostikeinrichtung nach Anspruch 3, **dadurch gekennzeichnet**, daß die Sensorelemente (75 bis 77) an die Außenkanten der unterschiedlichen Filmkassettenformate (A, B, C) abtastenden Stellen der Halterung (44) angeordnet sind.

5. Röntgendiagnostikeinrichtung nach Anspruch 4, **dadurch gekennzeichnet**, daß für jedes Filmkassettenformat (A, B, C) jeweils ein Paar die Kassettenbreite bestimmende Sensorelemente (75, 76, 77) vorhanden sind, die für gleiche Kassettenhöhe in gleicher, für unterschiedliche Kassettenhöhe in verschiedener Ebene (I, II) an der Halterung (44) angeordnet sind.

6. Röntgendiagnostikeinrichtung nach Anspruch 1, **dadurch gekennzeichnet**, daß die Sensoranordnung wenigstens einen schwenkbar gehalterten Tasthebel (63, 64; 70) enthält, welcher mit seinem freien Ende an einer das Format der Filmkassette (45) bestimmenden Referenzfläche (66, 67, 72) anliegt und mit den Tasthebeln Winkelgeber (61, 62, 71, 73) verbunden sind, welche den Schwenkstellungen (α, β, γ) der Tasthebel entsprechende Signale liefern.

7. Röntgendiagnostikeinrichtung nach Anspruch 6, **dadurch gekennzeichnet**, daß als Winkelgeber im Schwenkpunkt der Tasthebel (63, 64, 70) angeordnete Potentiometer (61, 62, 71) vorgesehen sind.

8. Röntgendiagnostikeinrichtung nach Anspruch 7, **dadurch gekennzeichnet**, daß der Filmkassettenhalter eine vertikal ausgerichtete Platte (44) enthält, gegen die die Filmkassette (45) anlegbar ist, daß auf der Vorderseite der Platte ein erster Tasthebel (64) vorhanden ist, welcher mit seinem freien Ende gegen eine erste Referenzfläche (67) der Filmkassette (45) anliegt und daß auf der Rückseite der Platte ein zweiter Tasthebel (63) angeordnet ist, welcher mit einem durch einen Schlitz (68) der Platte hindurchgreifenden Abtastelement (65, 47) gegen eine zweite Referenzfläche (66) der Filmkassette anliegt.

9. Röntgendiagnostikeinrichtung nach Anspruch 8, **dadurch gekennzeichnet**, daß die Schwenklager (42, 43; 59, 60) der beiden Tasthebel (63, 64) gleichsam die mechanische Halterung der Platte (44) bilden.

10. Röntgendiagnostikeinrichtung nach Anspruch 9, **dadurch gekennzeichnet**, daß die Tasthebel (63, 64) mit Übertragungsstangen (59, 60) winkelsteif verbunden sind, die in die Filmkassettenhalterung (44) tragenden Rohren (42, 43) drehbar gelagert sind und an ihren freien Enden mit den Winkelgebern (61, 62) verbunden sind.

## Claims

1. Dental X-ray diagnostic device for providing, on the one hand, panoramic tomographs of the jaw and, on the other hand, teleradiographs of the skull of a patient, containing a first film cassette holding support (5), which is close to the subject, for receiving film cassettes (11) for providing the panoramic tomographs and a second film cassette holding support (44), which is remote from the subject, for receiving film cassettes (45) for providing the teleradiographs, with the first film cassette holding support (5) being formed in such a way that in the case of teleradiographs it can be brought out of the beam path of the radiation source (16), and containing, furthermore, a holding support (20) for a diaphragm, provided between radiation source (16) and subject, for limiting the radiation beam, which diaphragm is supported such that for the purpose of changing the film cassette format (A, B, C) it can be exchanged for another with a different diaphragm opening, characterised in that the second film cassette holding support (44) has a sensor arrangement (59 to 65; 70, 71, 73; 75 to 77) which detects the position and the format (A, B, C) of the film cassette (45) inserted into the holding support (44), in that the diaphragm limiting the radiation beam is formed as a multiple diaphragm part (17) with several diaphragm openings (24 to 26), in that the multiple diaphragm part (17) can be controlled by means of an adjusting device (14) so that the diaphragm openings can be aligned, in each case, with the beam path of the radiation source, and in that a logic circuit arrangement (80) is provided, which arrangement processes the signals obtained from the sensor arrangement and supplies them to a control unit (81, 86) which controls the adjusting device (14) for the multiple diaphragm part (17) as a function of the selected film format.

2. X-ray diagnostic device according to claim 1, characterised in that a further sensor arrangement (34, 35) is present at the multiple diaphragm part (17), which arrangement delivers signals which correspond to the position of the individual diaphragms (21 to 23) and are supplied to a comparison logic unit (85) into which the signals from the sensor arrangement (61, 62; 70, 71; 75 to 77) determining the film cassette format are also input, and in that the radiation source (16) is controlled with the output signal of the comparison logic unit (85).

3. X-ray diagnostic device according to claim 1, characterised in that a sensor arrangement with optical elements (70, 71; 73, 75 to 77), such as, for example, light-emitting diodes and photoelements or reflex light barriers, is provided and the elements are arranged on reference surfaces (66, 67) of the film cassette holding support (44), which surfaces determine the width and/or height of the film cassette format (A, B, C).

4. X-ray diagnostic device according to claim 3, characterised in that the sensor elements (75 to 77) are arranged at points of the holding support (44) scanning the outer edges of the different film cassette formats (A, B, C).

5. X-ray diagnostic device according to claim 4, characterised in that for each film cassette format (A, B, C) there is, in each case, a pair of sensor elements (75, 76, 77) which determine the cassette width and which for the same cassette height are arranged on the support (44) in the same plane and for a differing cassette height are arranged on the support (44) in a different plane (I, II).

6. X-ray diagnostic device according to claim 1, characterised in that the sensor arrangement contains at least one sensing lever (63, 64; 70) which is supported such that it can be swung and which rests with its free end against a reference surface (66, 67 72) determining the format of the film cassette (45), and connected with the sensing levers there are angle transmitters (61, 62, 71, 73) which deliver signals corresponding to the positions of swing (α, β, γ) of the sensing levers.

7. X-ray diagnostic device according to claim 6, characterised in that potentiometers (61, 62, 71) are provided as angle transmitters arranged at the point of swing of the sensing levers (63, 64, 70).

8. X-ray diagnostic device according to claim 7, characterised in that the film cassette holder contains a vertically aligned plate (44) against which the film cassette (45) can be laid, in that a first sensing lever (64) is present on the front side of the plate, which lever rests with its free end against a first reference surface (67) of the film cassette (45) and in that arranged on the rear side of the plate there is a second sensing lever (63) which rests with a scanning element (65, 47), engaging through a slot (68) of the plate, against a second reference surface (66) of the film cassette.

9. X-ray diagnostic device according to claim 8, characterised in that the swing bearings (42, 43; 59, 60) of the two sensing levers (63, 64) substantially form the mechanical holding support of the plate (44).

10. X-ray diagnostic device according to claim 9, characterised in that the sensing levers (63, 64) are connected with transmission rods (59, 60) which are at a rigid angle, are rotatably mounted in tubes (42, 43) carrying the film cassette holding support (44) and are connected at their free ends with the angle transmitters (61, 62).

## Revendications

1. Installation de radiodiagnostic dentaire pour l'établissement d'une part de tomographies panoramiques de la mâchoire et d'autre part de téléradiographies du crâne d'un patient, comportant un premier support (5) de cassettes à film, proche de l'objet et servant à loger des cassettes à film (11) pour l'établissement des tomographies panoramiques, et un second support (44) de cassettes à film, éloigné de l'objet et servant à loger des cassettes à film (45) pour l'établissement des téléradiographies, et dans lequel le premier support (5) de cassettes à film est agencé de telle sorte que lors de téléradiographies, il peut être amené hors du trajet de rayonnement de la source de rayonnement (16), et comportant en outre un support (20) pour un diaphragme disposé entre la source de rayonnement (16) et l'objet et servant à limiter le faisceau de rayonnement, et qui est maintenu de manière à pouvoir être remplacé par un autre diaphragme possédant une autre ouverture, dans le but de modifier le format (A,B,C) des cassettes à film, caractérisé par le fait que le second support (44) de cassettes à film possède un dispositif de détection (59 à 65; 70, 71, 73; 75 à 77), qui détecte la position et le format (A,B,C) de la cassette à film (45) insérée dans le support (44), que le diaphragme, qui limite le faisceau de rayonnement, est agencé sous la forme d'un bloc de diaphragmes multiples (17) comportant plusieurs ouvertures (24 à 26), que le bloc à diaphragmes multiples (17) peut être commandé au moyen d'un dispositif de réglage (14) de manière que les ouvertures de diaphragme peuvent être alignées respectivement avec le trajet de rayonnement d'une source de rayonnement, et qu'il est prévu un circuit logique (80), qui prépare des signaux obtenus par le dispositif de détection et les envoie à une l'unité de commande (81, 86), qui commande le dispositif de réglage (14) pour le bloc à diaphragmes multiples (17) en fonction du format de film qui est choisi.

2. Installation de radiodiagnostic suivant la revendication 1, caractérisée par le fait qu'il est prévu, sur le bloc à diaphragmes multiples (17), un autre dispositif de détection (34, 35), qui fournit des signaux qui correspondent à la position des différents diaphragmes (21 à 23) et qui sont envoyés à une unité logique de comparaison (85), dans laquelle sont également introduits les signaux provenant du dispositif de détection (61, 62; 70, 71; 75 à 77) qui déterminent le format de la cassette à film, et que la source de rayonnement (16) est commandée par le signal de sortie de l'unité logique de comparaison (85).

3. Installation de radiodiagnostic suivant la revendication 1, caractérisée par le fait qu'il est prévu un dispositif de détection comportant des éléments optiques (70, 71; 73, 75 à 77), comme par exemple des diodes à luminescence et des éléments photoélectriques ou des relais photoélectriques à réflexion, et que les éléments sont disposés sur des surfaces de référence (66, 67) du support (44) de cassettes à film, qui déterminent la largeur et/ou la hauteur du format (A, B, C) des cassettes à film.

4. Installation de radiodiagnostic suivant la revendication 3, caractérisée par le fait que les éléments de détection (75 à 77) sont disposés sur les bords extérieurs des points du support (44), qui explorent différents formats (A,B,C) de cassettes à film.

5. Installation de radiodiagnostic suivant la revendication 4, caractérisée par le fait que pour chaque format (A,B,C) de cassettes à film, il est prévu respectivement un couple d'éléments de détection (75, 76, 77), qui déterminent la largeur de la cassette et qui sont disposés, pour la même hauteur de cassette, dans un même plan (I) et, pour une hauteur différente de cassette, dans un plan différent (II), sur le support (44).

6. Installation de radiodiagnostic suivant la revendication 1, caractérisée par le fait que le dispositif de détection contient au moins un levier de palpage (63, 64; 70), qui est monté pivotant et qui s'applique par son extrémité libre contre une surface de référence (66, 67, 72) déterminant le format de la cassette à film (45), et qu'aux leviers de palpage sont raccordés des transmetteurs d'angles (61, 62, 71, 73), qui envoient des signaux correspondant aux positions en rotation (α, β, γ) des leviers de palpage.

7. Installation de radiodiagnostic suivant la revendication 6, caractérisée par le fait qu'il est prévu comme transmetteurs d'angle, des potentiomètres (61, 62, 71) disposés au centre de gravité des leviers de palpage (63, 64, 70).

8. Dispositif de radiodiagnostic suivant la revendication 7, caractérisé par le fait que le support de cassettes à film contient une plaque verticale (44), contre laquelle la cassette à film (45) peut être appliquée, que sur la face avant de la plaque il est prévu un premier levier de palpage (64), qui s'applique par son extrémité libre contre une première surface de référence (67) de la cassette à film (45) et que sur la face arrière de la plaque est disposé un second levier de palpage (63), qui s'applique par un élément de palpage (65, 47), qui traverse une fente (68) de la plaque, contre une seconde surface de référence (66) de la cassette à film.

9. Installation de radiodiagnostic suivant la revendication 8, caractérisée par le fait que les paliers (42, 43; 59, 60) des deux leviers de palpage (63, 64) forment pour ainsi dire le support mécanique de la plaque (44).

10. Installation de radiodiagnostic suivant la revendication 9, caractérisée par le fait que les leviers de palpage (63, 64) sont raccordés, selon un angle fixe, à des barres de transmission (59,60), qui sont montées tournantes dans des tubes (42, 43), qui portent le support (44) de cassettes à film et qui sont reliées, par leurs extrémités libres, aux transmetteurs d'angle (61, 62).
